(19) Europäisches Patentamt
European Patent Office
Office européen des brevets

(11) Publication number: **0 107 216**
**B1**

(12) **EUROPEAN PATENT SPECIFICATION**

(45) Date of publication of patent specification: **01.10.86**

(21) Application number: **83201306.4**

(22) Date of filing: **12.09.83**

(51) Int. Cl.$^4$: **C 07 D 249/18,** C 07 D 235/08, A 01 N 43/52, A 01 N 43/64

(54) **Heterocyclic compounds, their preparation, compositions containing them and method of controlling unwanted plant growth.**

(30) Priority: **20.09.82 US 419860**
**20.09.82 US 419865**

(43) Date of publication of application:
**02.05.84 Bulletin 84/18**

(45) Publication of the grant of the patent:
**01.10.86 Bulletin 86/40**

(84) Designated Contracting States:
**AT BE CH DE FR GB IT LI LU NL SE**

(56) References cited:
**US-A-3 856 812**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Pilgram, Kurt Hans Gerhard**
**2607 Warwick Lane**
**Modesto, CA 95350 (US)**
Inventor: **Skiles, Richard Daniel**
**2912 Debbie Lane**
**Modesto, CA 95350 (US)**

(74) Representative: **Hunter, Keith Roger Ian et al**
**4 York Road**
**London SE1 7NA (GB)**

**EP 0 107 216 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This application relates to novel heterocylic compounds, their preparation, herbicidal compositions containing them and to a method of controlling unwanted plant growth using them.

In US—A—3856812 phenoxyacetic acid derivatives of formula

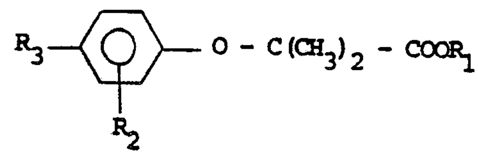

are described, in which $R_1$ is hydrogen and $C_{1-10}$ alkyl, $R_2$ is hydrogen, halogen or a $C_{1-4}$ alkyl group and $R_3$ may be an optionally substituted 1-benzimidazolyl or 1-benzotriazolyl group. These derivatives have cholesterol and triglyceride blood serum level-lowering and enzyme-inducing activities. They are therefore employed as drugs or may be used in the preparation of other drugs.

It has now been found that other 4-(benz(otri/imid)azol-1-yl) phenoxy-alkanoic acids, esters and salts adversely effect the growth of plants to which they are applied, and thus are of interest for controlling the growth of unwanted plants. These compounds are described by the formula:

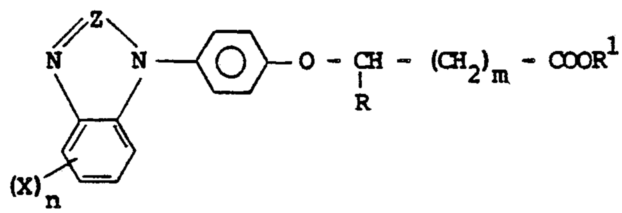

(I)

wherein Z is nitrogen or $CR^3$; X is halogen and n is 0, 1 or 2, or n is 1 and X is cyano, methyl, halomethyl, methoxy, halomethoxy, methylthio, halomethylthio, methylsulfinyl, halomethylsulfinyl, methylsulfonyl or halomethylsulfonyl; R is methyl, ethyl or methoxymethyl; m is 0, 1 or 2; $R^1$ is hydrogen, alkyl of one to four carbon atoms, an alkali metal or ammonium $(N(R^2)_4)$ ion, wherein each $R^2$ independently is hydrogen or alkyl of one to six carbon atoms; and $R^3$ is hydrogen, methyl or ethyl.

In these compounds, "halogen" and "halo" designate halogen selected from bromine, chlorine and fluorine — fluorine being generally preferable; "halomethyl", in each case, includes mono-, di- and trihalomethyl moieties.

Preferably n = 1 and X is selected from fluoro and trifluoromethyl, the latter being particularly preferred.

If n does not equal 0, the substituent(s) X may be bonded to the benzimidazole or benzotriazole moiety at the 4, 5, 6 or 7-position. The best results are obtained when n = 0, 1 or 2, and (one) X is bonded to the benzimidazole or benzotriazole moiety at the 5-position.

The group represented by R preferably is methyl or ethyl, particularly methyl. $R^1$ preferably represents hydrogen, methyl or ethyl. The acids appear to have the best herbicidal activity, and $R^1$ is particularly hydrogen therefore. The benzotriazoles are preferred; so Z especially represents a nitrogen atom.

Highest phytotoxicity appears to reside in the compounds according to Formula I wherein n is one, m is zero, X represents the trifluoromethyl moiety, bonded to the ring at the 5-position, Z is nitrogen, R represents methyl or ethyl, and $R^1$ represents hydrogen, methyl or ethyl, so that this subclass forms a particularly preferred aspect of the invention.

The invention also provides a process for the preparation of a compound according to the invention, characterized in that a compound of the formula

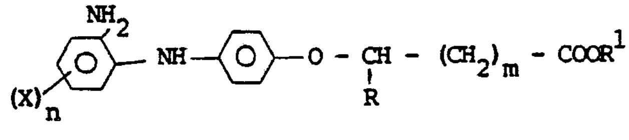

(II)

wherein n, m, X, R and $R^1$ have the meanings defined above, is subjected to a cyclisation reaction.

Such reactions are known per se, and may be carried out in any suitable way known in the art to produce imidazole or triazole rings.

In particular the invention relates to a cyclisation process, wherein the compound of formula II is converted either to a compound according to formula I wherein Z = N, by subjecting it to a diazotization reaction, or to a compound according to formula I wherein Z = $CR^3$, by subjecting it to a condensation reaction with an orthoalkanoate of formula $R^3C(OR^4)_3$, wherein $R^3$ has the meaning defined above and $R^4$ is an alkyl group of one to four carbon atoms, and each reaction being followed by a conversion into another compound according to formula I, if desired.

In particular is the diazotization reaction carried out by reacting with an alkali metal nitrite, e.g. $NaNO_2$, in the presence of an aqueous mineral acid, e.g. HCl or $H_2SO_4$. Most preferably one treats an acid solution or suspension of the compound of formula II, wherein $R^1$ is a $C_{1-4}$ alkyl group especially, at a temperature below 25°C, e.g. at −5 to 10°C, with $NaNO_2$.

The condensation reaction is conveniently effected by heating the compound of formula II, wherein $R^1$ is a $C_{1-4}$ alkyl group especially, with an excess of the orthoalkanoate, the latter being used as solvent as well as reactant.

The compounds of formula II may be prepared by reduction of the corresponding nitrophenyl compounds of formula:

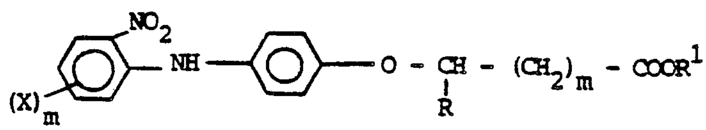

(III)

The reduction is conveniently effected by treatment of a solution of the nitro compound in an inert solvent such as tetrahydrofuran with hydrogen under pressure of a catalyst, such as charcoal impregnated with 10%w of palladium, using a suitable apparatus, such as a Parr shaker.

The nitro compounds of Formula III, may be prepared by methods known per se. The following two general methods may be used to prepare compounds of Formula III, for example:

(1) Treatment of a substituted phenol of formula

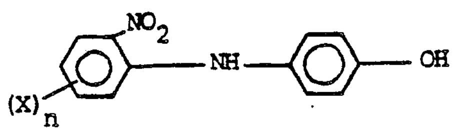

(IV)

with a haloalkanoic acid, ester or salt of formula

$$\text{halogen-}\underset{\displaystyle R}{\underset{|}{CH}}-(CH_2)_m-COOR^1 \qquad (V)$$

and

(2) Treatment of nitrohalobenzene of formula

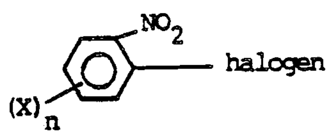

(VI)

with a substituted aniline of formula

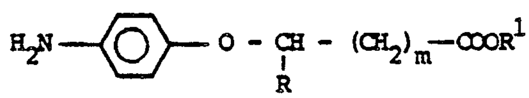

(VII)

In the case where m = 2, an alternative method of preparing the precursor of formula II is to prepare the alkenoic acid counterpart first, and then to hydrogenate catalytically, both saturating the double bond and reducing the nitro moiety to the amino moiety.

Conversion of an acid ($R^1$ = H) to an ester ($R^1$ = alkyl) or a salt, and vice versa, is effected by conventional methods.

As mentioned above, the compounds of the invention are of interest as herbicides. Accordingly, the invention includes the use of compound according to the invention as a herbicide, and a herbicidal composition, which comprises a compound according to formula I, together with a carrier. Furthermore the invention relates to a method of controlling unwanted plant growth, at a locus, which comprises treating the locus with a compound or a composition according to the invention. Application may be preemergence or post-emergence. The dosage of active ingredient used may, for example, be from 0.1 to 10.0 kg/ha.

A carrier in a composition according to the invention is any material with which the active ingredient is formulated to facilitate application to the locus to be treated, or to facilitate storage, transport or handling. A carrier may be a solid or a liquid, including a material which is normally gaseous but which has been compressed to form a liquid, and any of the carriers normally used in formulating agricultural

compositions may be used. Preferably compositions according to the invention contain 0.5 to 95% by weight of active ingredient.

Suitable solid carriers include natural and synthetic clays and silicates, for example natural silicas such as diatomaceous earths; magnesium silicate, for example talcs; magnesium aluminium silicates, for example attapulgites and vermiculites; aluminium silicates, for example kaolinites, montmorillonites and micas; calcium carbonate; calcium sulphate, ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; and chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, or sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrate, emulsions, suspension concentrates and aerosols. Wettable powders usually contain 25, 50 or 75% w of active ingredient and usually contain in addition to solid inert carrier, 3—10% w of stabiliser(s) and/or other additives such as penetrants or stickers. Dusts are usually formulated as dust concentrate having a similar composition to that of a wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by aggolomeration or impregnation techniques. Generally, granules will contain ½—75% w active ingredient and 0—10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10—50% w/v active ingredient, 2—20% w/v emulsifiers and 0—20% of other additives such as stabilisers, penetrants and corrosion inhibitors. Suspension concentrates are usually compounded so as to obtain a stable, non-sedimenting flowable product and usually contain 10—75% w of suspending agents such as protective colloids and thixotropic agents, 0—10% w of other additives such as defoamers, corrosion inhbibitors, stabilisers, penetrants and stickers, and water or an organic liquid in which the active ingredient is substantially insoluble; certain organic solids of inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The compositions of the invention may also contain other ingredients, for example, other compounds possessing herbicidal, insecticidal or fungicidal properties.

The following Examples illustrate the invention. In each case, the identity of the product, and of any intermediate involved, was confirmed by appropriate chemical and spectral analyses.

## Example 1

Ethyl 2-(4-(5-(trifluoromethyl)benzotriazol-1-yl)phenoxy)-propionate (1)

A mixture of 222.5 g of 4-chloro-3-nitrobenzotrifluoride and 109 g of p-aminophenol in 500 ml of isopropyl alcohol was refluxed for 8 hours. Then 101 g of triethylamine was added, the mixture was refluxed for 2.5 hours, then concentrated to dryness. The residue was treated with 1000 ml of ether. The

ethereal phase was separated, washed with water and diluted with 600 ml of hexane. The resulting mixture was filtered through silica gel and the filtrate was concentrated to give 4'-hydroxy-2-nitro-4-(trifluoromethyl)diphenylamine (*1A*), as a red solid, m.p.: 130—133°C.

A mixture of 29.8 g of *1A*, 18.4 g of ethyl 2-bromopropionate, 14 g of anhydrous potassium carbonate and 300 ml of 2-butanone was refluxed for 6 hours, poured into water, and the resulting mixture was extracted with ether. The extract was dried ($MgSO_4$), filtered and concentrated. Recrystallization of the residue from either/hexane gave ethyl 2-(4-(2-nitro-4-(trifluoromethyl)phenylamino)phenoxy)propionate (1B), as a crystalline solid, m.p.: 61—63°C. *1B*, in tetrahydrofuran, was treated with hydrogen (2.8 atm pressure in a Parr-shaker, 10% palladium-on-carbon catalyst), to give ethyl 2-(4-(2-amino-4-(trifluoromethyl)phenylamino)phenoxy) propionate (*1C*), as a solid, m.p.: 78°C.

A solution of 2.1 g of sodium nitrite in 25 ml of water was added drop-by-drop over a 5-minute period to a stirred solution of 7.4 g of *1C* in 150 ml of 12% hydrochloric acid, at 0°C. The mixture then was allowed to warm to room temperature and was stirred for 24 hours. It then was diluted with 200 ml of water and extracted with ether. The ether extract was dried ($MgSO_4$), filtered and concentrated to dryness. The residue was adsorbed on silica gel and chromatographed, using as eluent a 2:15:33 v/v/v mixture of tetrahydrofuran, ethyl acetate and hexane (hereinafter, Solvent A), to give *1*, as an amber syrup. *1B* also was prepared by an alternative method, as follows: A mixture of 28 g of 4-nitrophenol, 36 g of ethyl 2-bromopropionate, 27.6 g of potassium carbonate and 500 ml of butanone was stirred and refluxed for 17 hours. The resulting mixture was diluted with 500 ml of water and extracted with ether. The extract was concentrated in a rotary evaporator and the residue was recrystallized from ether-hexane to give ethyl 2-(4-nitrophenoxy) propionate (*1D*), as a yellow solid, m.p.: 55—56°C.

Hydrogenation of *1D* (as described in preparation of *1C*) gave ethyl 2-(4-aminophenoxy) propionate (*1E*), as a viscous oil.

A solution of 209 g of *1E*, 111 g of triethylamine and 225.5 g of 4-chloro-3-nitrobenzotrifluoride in 600 ml of 2-butanone was refluxed for 24 hours, then concentrated in a rotary evaporator. The oily residue was dissolved in 1200 ml of ether; the solution was washed with water, dried ($MgSO_4$) and concentrated. Purification by silica gel chromatography (eluent: Solvent A) gave *1B*.

Example 2

2-(4-(5-(trifluoromethyl)benzotriazol-1-yl)phenoxy)propionic acid (2)

A solution of 2 g of potassium hydroxide in 2 ml of water was added to a solution of 4.2 g of *1* in 25 ml of ethanol. The resulting mixture was heated on a steam bath for 15 minutes, then the ethanol was evaporated in a rotary evaporator. The residue was dissolved in 25 ml of water; the resulting solution was acidified with hydrochloric acid and extracted with ether. The extract was dried ($MgSO_4$), filtered and concentrated. The residual solid was recrystallized from ether-hexane to give *2*, as an off-white solid, m.p.: 122—125°C.

Example 3

Ethyl 2-(4-(5-(trifluoromethyl)benzotriazol-1-yl)phenoxy)butanoate (3)

A mixture of 14.9 g of *1A*, 14.8 g of ethyl 2-bromobutyrate, 18.8 g of potassium carbonate and 150 ml of butanone was stirred and refluxed for 8 hours, diluted with water, acidified with hydrochloric acid and extracted with ether. The extract was dried ($MgSO_4$) filtered and concentrated in a rotary evaporator, to give ethyl 2-(4-(2-nitro-4-(trifluoromethyl)phenylamino)phenoxy)butyrate (*3A*), as a viscous oil.

Hydrogenation of *3A* as described in Example 1 gave ethyl 2-(4-(2-amino-4-(trifluoromethyl)phenylamino)phenoxy)butyrate (*3B*), as a viscous oil.

A solution of 2.1 g of sodium nitrite in 10 ml of water was added drop-by-drop to a stirred solution of 5.0 g of *3B* in 150 ml of 12% hydrochloric acid at 5°C. The resulting mixture was stirred at room temperature for 2 days, diluted with 100 ml of water and extracted with ether. The ether was evaporated from the extract and the residue was chromatographed over silica gel, using as eluent a 20/4/1 v/v/v mixture of hexane, ethyl acetate and tetrahydrofuran, to give *3*, as a reddish syrupy liquid.

Example 4

2-(4-(5-trifluoromethyl)benzotriazol-1-yl)phenoxy)butanoic acid (4)

The ester prepared in Example 3 was hydrolysed by a procedure analogous to the one described in Example 2, yielding an off-white solid of melting point 122—125°C.

Example 5

Methyl 3-methoxy-2-(4-(5-trifluoromethyl)benzotriazol-1-yl)-phenoxy)propionate (5)

480 g of mercuric acetate was added to a solution of 139 g of methyl acrylate in 170 ml of methanol. The resulting mixture was stirred for 3 days at room temperature. To the mixture, cooled in an ice bath, a solution of 100 g of potassium bromide in 600 ml of water was added. A heavy oil formed, was separated from the mixture and was extracted with chloroform. The extract was washed with water, dried ($MgSO_4$) and filtered. The filtrate was heated to 60°C and 36.9 g of bromine was added, drop-by-drop over a 2-hour period. The resulting mixture was cooled to 5°C and concentrated in a rotary evaporator. The liquid residue was distilled to give methyl 2-bromo-3-methoxypropionate (*5A*), b.p.: 69—72°C (3—5 Torr.).

A mixture of 69 g of *5A*, and 104 g of *1A*, 45 g of potassium carbonate and 1000 ml of 2-butanone was stirred and refluxed for 3 days. The product, methyl 2-(4-(2-nitro-4-(trifluoromethyl)-3-methoxy propionate (*5B*), was obtained as solid, m.p.: 57°C, by procedures described for the isolation of *1B*.

A solution of 17 g of *5B* in 100 ml of tetrahydrofuran was treated with hydrogen (2.8 atm) hydrogen pressure, Parr-Shaker, 10% palladium-on-charcoal catalyst) to give methyl 2-(4-(2-amino-4-(trifluoromethyl)phenylamino)phenoxy)-3-methoxypropionate (*5C*), as an oil. A mixture of 10.5 g of *5C* and 50 ml of concentrated hydrochloric acid was warmed to 60°C and while it was being cooled to 5°C, a solution of 2.1 g of sodium nitrite in 10 ml of water was added, drop-by-drop. The resulting mixture was stirred at room temperature for 18 hours, then was extracted with ether. The extract was concentrated and the residue was chromatographed over silica gel to give *5*, as an offwhite solid, m.p.: 89—92°C.

Example 6

3-methoxy-2-(4-(5-(trifluoromethyl)benzotriazol-1-yl)-phenoxy)propionic acid (6)

By a procedure analogous to Example 2, the compound *5* was converted into the corresponding acid (*6*), a colorless solid, m.p.: 139—142°C.

Example 7

2-(4-(5-fluorobenzotriazol-1-yl)phenoxy)propionic acid (7)

2.5 g of sodium hydride paste (60% in mineral oil) was added in portions to a stirred solution of 9.9 g of 2,5-difluoronitrobenzene and 12.7 of *1E* in 200 ml of dimethyl sulfoxide. The resulting mixture was held at 160—170°C for 24 hours, then was cooled, diluted with 1000 ml of water and extracted with ether. The extract was chromatographed on silica gel to give ethyl 2-(4-(4-fluoro-2-nitrophenylamino)phenoxy)propionate (*7A*) as a viscous oil.

A solution of 13.0 g of *7A* in 150 ml of tetrahydrofuran containing 3.7 g of 10% palladium-on-charcoal catalyst was hydrogenated on a Parr-shaker at 2.8 atm hydrogen pressure. After 3 hours, the mixture was filtered and the filtrate was concentrated under reduced pressure. The residual oil was ethyl 2-(4-(2-amino-4-fluorophenylamino)phenoxy)propionate (*7B*).

A solution of 2.1 g of sodium nitrite in 25 ml of water was added drop-by-drop to a stirred solution of 6.7 g of *7B* in 150 ml of 12% hydrochloric acid at 2°C. The resulting mixture was stirred at room temperature for 12 hours, then was filtered. Recrystallization of the filter cake from ether/hexane gave *7*, as a solid, m.p.: 164—166°C.

Example 8

Ethyl 2-(4-(5-trifluoromethyl)benzimidazol-1-yl-phenoxy)-propionate (8)

5.3 g of *1C* was dissolved in 50 ml of triethyl orthoformate, the solution was refluxed for 1 hour, then concentrated under reduced pressure. The residue, an oil, was chromatographed over silica gel, using as eluent Solvent A, to give *8*, as a viscous liquid.

Example 9

2-(4-(5-(trifluoromethyl)benzimidazol-1-yl)phenoxy)-propionic acid (9)

A mixture of 3.7 g of *8*, 0.39 g of sodium hydroxide and 50 ml of ethanol was refluxed for two hours, then it was cooled to room temperature, acidified with hydrochloric acid and extracted with ether. Concentration of the ether extract gave, *9*, as a white solid, m.p.: 181—182°C.

Examples 10 and 11

Ethyl 2-4-(2-methyl-5-(trifluoromethyl)benzimidazol-1-yl)phenoxy)propionate (10).

Ethyl 2-(4-(2-ethyl-5-(trifluoromethyl)benzimidazol-1-yl)phenoxy)propionate (11)

*10* was prepared, as an oil, from *1C* and triethyl orthoacetate, and *11* was prepared, as a solid, m.p.: 66—68°C, from *1C* and triethyl orthopropionate by the procedure described in Example *8*.

Example 12

2-(4-(2-methyl-5-(trifluoromethyl)benzimidazol-1-yl)phenoxy)-propionic acid (12)

*12* was prepared, as a solid, m.p.: 107°C, from *10* by the procedure described in Example 9.

Example 13

Ethyl 2-(4-(2-methyl-5-(trifluoromethyl)benzimidazol-1-yl)-phenoxy)butyrate (13)

A mixture of 14.9 g of *1A*, 14.8 g of ethyl 2-bromobutyrate, 18.8 g of potassium carbonate and 150 ml of butanone was stirred and refluxed for 8 hours. The mixture then was diluted with water, acidified with hydrochloric acid and extracted with ether. The ether extract was dried ($MgSO_4$), filtered and concentrated in a rotary evaporator to give ethyl 2-(4-(2-nitro-4-(trifluoromethyl)phenylamino)-phenoxy)butyrate (*13A*), as a viscous oil.

Hydrogenation of 13A as described in Example 1 gave ethyl 2-(4-(2-amino-4-(trifluoromethyl)phenylamino)phenoxy)-butyrate (*13B*), as a viscous oil.

A solution of 7 g of *13B* in 50 ml of triethyl orthoacetate was heated at 140°C for 2 hours. The resulting

mixture was cooled to room temperature, absorbed on silica gel and chromatographed, using the eluent described in Example 1, to give *13* as a viscous oil.

Example 14

Ethyl 2-(4-(5-trifluoromethyl)benzimidazol-1-yl)phenoxy)butyrate

*14* was prepared, as an oil, from *13B* and triethyl orthoformate, by the procedure described for the preparation of *13* from *13B*.

Example 15

2-(4-(2-methyl-5-(trifluoromethyl)benzimidazol-1-yl)phenoxy)butyric acid (15)

The ester prepared in Example 13 was hydrolysed by the procedure described in Example 9, yielding *15*, as a white solid, m.p.: 98°C.

Example 16

2-(4-(5-(trifluoromethyl)benzimidazol-1-yl)phenoxy)butyric acid (16)

*16* was prepared, as a solid, m.p.: 214—215°C, from *14*, by the procedure described in Example 9.

Example 17

Methyl 2-(4-(2-methyl-5-(trifluoromethyl)benzimidazol-1-yl)phenoxy)-3-methoxypropionate (17)

A solution of 7 g of *5C* in 25 ml of triethyl orthoacetate was refluxed for 4 hours. The resulting mixture was concentrated in a rotary evaporator and chromatographed over silica gel to give *17*, as a viscous oil.

Example 18

2-(4-(2-methyl-5-(trifluoromethyl)benzimidazol-1-yl)-phenoxy)-3-methoxypropionic acid (18)

The ester *17* was hydrolysed to the acid *18* by the procedure of Example 2, to give a light tan crystalline solid, m.p.: 111—112°C.

Example 19

Ethyl 4-(4-(2-methyl-5-(trifluoromethyl)benzimidazol-1-yl)-phenoxy)pentanoate (19)

A mixture of 56.2 g of trans-2-pentenoic acid and 100 g of N-bromosuccinimide in 1000 ml of carbon tetrachloride was stirred and refluxed for 24 hours. The resulting mixture was cooled to room temperature and filtered. The filtrate was concentrated to a volume of about 300 ml, cooled to room temperature and filtered to give trans-4-bromo-2-pentenoic acid, as a white crystalline solid (*19A*), m.p.: 78—80°C.

25 g of hydrogen chloride was introduced into a solution of 139.2 g of *19A* in 1000 ml of ethanol. The resulting mixture was refluxed for 36 hours, concentrated in a rotary evaporator, mixed with ice water and extracted with ether. The extract was washed to neutrality with aqueous sodium bicarbonate, dried ($MgSO_4$) and concentrated. The liquid residue was fractionally distilled to give ethyl 4-bromo-2-pentenoate (*19B*), as a colorless liquid, b.p.: 82°C (1.8 Torr.).

A mixture of 71.5 g of *1A*, 45.0 g of *19B*, 33 g of potassium carbonate and 800 ml of 2-butanone was stirred and refluxed for 24 hours. The resulting mixture was divided in half. One half was concentrated to dryness, the residue was diluted with ether and shaken with 2.5% sodium hydroxide solution. The ether phase was separated, the ether was evaporated, and the residue was chromatographed over silica gel to give ethyl 4-(4-(2-nitro-4-(trifluoromethyl)phenylamino)phenoxy)-2-pentenoate (*19C*), as a viscous oil.

23 g of *19B* and 16.8 g of potassium carbonate were added to the second half of the above mixture, and the resulting mixture was refluxed for 8 hours. The mixture was concentrated to dryness, the residue was mixed with ether and water, warmed, then cooled to room temperature and acidified with hydrochloric acid. The ether phase was separated, concentrated and chromatographed over silica gel to give 4-(4-(2-nitro-4-(trifluoromethyl)phenylamino)phenoxy)-2-pentenoic acid (*19D*), as a viscous oil.

*19D*, in solution in tetrahydrofuran was placed on a Parr shaker and treated with hydrogen (2.8 atm hydrogen pressure, Parr shaker, 10% palladium-on-charcoal catalyst) to give 4-(4-(2-amino-4-(trifluoromethyl)phenylamino)phenoxy)pentanoic acid (*19E*), as an oil.

A solution of 3.1 g of *19E* in 50 ml of triethyl orthoacetate was refluxed for 2 hours. The resulting mixture was concentrated in a rotary evaporator and the residue was chromatographed over silica gel to give *19*, as a viscous oil.

Example 20

4-(4-(2-methyl-5-(trifluoromethyl)benzimidazol-1-yl)-phenoxy)pentanoic acid (20)

The ester *19* was hydrolysed by the procedure of Example 9 to give *20*, a a white solid, m.p.: 67—68°C.

Example 21

Ethyl 2-(4-(2-methyl-5-fluorobenzimidazol-1-yl)phenoxy)propionate (21)

A solution of 4.28 g of *7B* in 45 g of triethyl orthoacetate was refluxed for 2 hours. The resulting mixture was concentrated under reduced pressure and the residue was chromatographed over silica gel to give *21*, as a crystalline solid, m.p.: 69—71°C.

Example 22

*Herbicidal Activity*

In the following experiments, the species of plants that were tested were:

BG — Barnyardgrass (watergrass) — *Echinochloa crus-galli*
CG — Large crabgrass — *Digitaria sanguinalis*
DB — Downy brome — *Bromus tectorum*
YF — Yellow foxtail — *Setaria lutescens*
RP — Redroot pigweed — *Amaranthus retroflexus*
SP — Sicklepod — *Cassia obtusifolia*
VL — Velvetleaf — *Abutilon theophrasti*
GC — Garden cress — *Lepidium Sativum*
JG — Johnsongrass — *Sorghum halepense*

*Test Procedures*

The preemergence (soil) herbicidal activity of compounds of the invention was evaluated by planting seeds of barnyardgrass, garden cress, downy brome, velvetleaf, yellow foxtail, and sicklepod in test tubes, nominally measuring 25 × 200 millimeters, filled about three quarters full of untreated soil, in each case covered on top with about 2.5 cubic centimeters of soil treated with a certain amount of the test compound. The treated soil applied to the tubes containing the barnyardgrass and cress seeds contained one milligram of the test compound per tube, and contained 0.1 milligram of the test compound per each tube containing the seeds of the other plants. The dosages were approximately 25 and 2.5 kg of test compound per hectare, respectively. The seeds were planted on top of the treated soil and covered with about 1.5 cubic centimeters of untreated soil. The planted soil was held under controlled conditions and temperature, moisture, and light for 9 to 10 days. The amounts of germination and growth in each tube were evaluated on the standard 0 to 9 scale, of the European Weed Research Council (EWRC).

The postemergence (foliar) herbicidal activity of compounds of the invention was evaluated by spraying 6-day-old Johnsongrass plants, 9-day-old velvetleaf plants, 9-day-old yellow foxtail plants and 9-day-old sicklepod plants to runoff with a liquid formulation of the test compound. The crabgrass and pigweed plants were sprayed with 2.4 milliliters of a 0.25% solution (about 11 kg/ha), and other plants were sprayed with 2.4 milliliters of a 0.025% solution (about 1.1 kg/ha). The sprayed plants were held under controlled conditions of temperature, moisture and light for 7 to 8 days and the effect of the test compound was then evaluated visually, the results being rated on the 0 to 9 scale described above.

Results of the preemergence and postemergence herbicidal activity tests conducted on the compounds of the invention are set forth in Table 1.

TABLE I

| Compound of Example | Preemergence | | | | | | Postemergence | | | | | |
|---|---|---|---|---|---|---|---|---|---|---|---|---|
| | BG | GC | DB | VL | YF | SP | CG | RP | JG | VL | YF | SP |
| *1* | 8 | 4 | 7 | 0 | 7 | 0 | 9 | 8 | 9 | 2 | 8 | 0 |
| *2* | 9 | 7 | 7 | 4 | 7 | 2 | 9 | 9 | 9 | 3 | 9 | 4 |
| *3* | 7 | 3 | 6 | 0 | 6 | 0 | 9 | 6 | 9 | 2 | 8 | 2 |
| *4* | 9 | 8 | 6 | 3 | 7 | 0 | 8 | 8 | 9 | 3 | 8 | 5 |
| *5* | 7 | 5 | 0 | 2 | 0 | 3 | 8 | 8 | 6 | 0 | 2 | 2 |
| *6* | 7 | 7 | 3 | 4 | 0 | 2 | 7 | 5 | 6 | 0 | 2 | 2 |
| *7* | 8 | 9 | 3 | 0 | 4 | 0 | 0 | 3 | 0 | 0 | 0 | 3 |
| *8* | 6 | 6 | 0 | 0 | 0 | 0 | 9 | 8 | 7 | 2 | 4 | 5 |
| *9* | 8 | 8 | 6 | 0 | 0 | 2 | 7 | 7 | 7 | 2 | 5 | 5 |
| *10* | 7 | 4 | 0 | 2 | 0 | 0 | 9 | 9 | 9 | 2 | 8 | 2 |
| *11* | 0 | 4 | 0 | 0 | 0 | 0 | 3 | 6 | 0 | 3 | 0 | 2 |
| *12* | 9 | 6 | 5 | 3 | 5 | 2 | 8 | 7 | 8 | 2 | 6 | 1 |
| *13* | 0 | 0 | 0 | 0 | 0 | 0 | 8 | 3 | 7 | 0 | 2 | 2 |

TABLE I (continued)

| Compound of Example | Preemergence BG | GC | DB | VL | YF | SP | Postemergence CG | RP | JG | VL | YF | SP |
|---|---|---|---|---|---|---|---|---|---|---|---|---|
| *14* | 0 | 5 | 0 | 0 | 0 | 0 | 7 | 6 | 3 | 2 | 2 | 2 |
| *15* | 7 | 7 | 0 | 0 | 0 | 0 | 8 | 4 | 7 | 2 | 2 | 2 |
| *16* | 7 | 8 | 0 | 0 | 0 | 0 | 1 | 2 | 1 | 0 | 0 | 2 |
| *17* | 4 | 4 | 2 | 0 | 2 | 0 | 4 | 9 | 2 | 2 | 2 | 0 |
| *18* | 0 | 2 | — | 0 | 0 | 0 | 3 | 5 | 0 | 3 | 0 | 0 |
| *19* | 0 | 6 | 0 | 0 | 0 | 0 | 2 | 4 | 0 | 0 | 0 | 0 |
| *20* | 0 | 0 | 0 | 0 | 0 | 0 | 5 | 9 | 4 | 0 | 0 | 0 |
| *21* | 0 | 0 | 0 | 0 | 0 | 0 | 6 | 8 | 1 | 3 | 0 | 2 |

**Claims for the Contracting States: BE CH DE FR GB IT LI LU NL SE**

1. A compound of the formula:

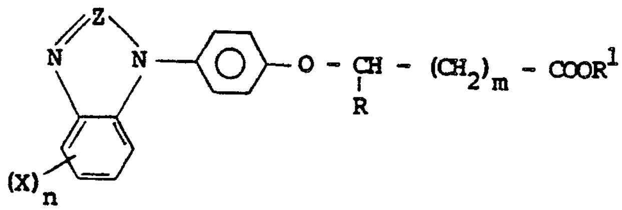

(I)

wherein Z is nitrogen or $CR^3$; X is halogen and n is 0, 1 or 2, or n is 1 and X is cyano, methyl, halomethyl, methoxy, halomethoxy, methylthio, halomethylthio, methylsulfinyl, halomethylsulfinyl, methylsulfonyl or halomethylsulfonyl; R is methyl, ethyl or methoxymethyl; m is 0, 1 or 2; $R^1$ is hydrogen, alkyl of one to four carbon atoms, an alkali metal or ammonium $(N(R^2)_4)$ ion, wherein each $R^2$ independently is hydrogen or alkyl of one to six carbon atoms; and $R^3$ is hydrogen, methyl or ethyl.

2. A compound according to claim 1, wherein n = 1 and X is selected from fluoro and trifluoromethyl.

3. A compound according to claim 1 or 2, wherein n = 1 or 2 and (one) X is bonded to the benzimidazole or benzotriazole moiety at the 5-position.

4. A compound according to anyone of claims 1 to 3, wherein R represents a methyl or ethyl group.

5. A compound according to any one of claims 1 to 4, wherein $R^1$ represents hydrogen, methyl or ethyl.

6. A compound according to any one of claims 1 to 5, wherein m = 0.

7. Process for preparing a compound of general formula I, characterised in that a compound of the formula,

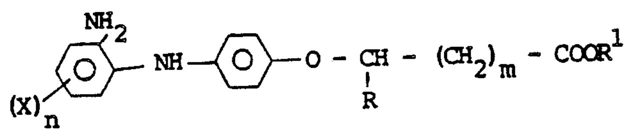

(II)

wherein n, X, R, and $R^1$ have the meanings defined in claim 1, is subjected to a cyclisation reaction.

8. A process as claimed in claim 7, wherein the compound of the formula II is converted either to a compound according to formula I wherein Z = N, by subjecting it to a diazotization reaction, or to a compound according to formula I wherein Z = $CR^3$, by subjecting it to a condensation reaction with an orthoalkanoate of formula $R^3C(OR^4)_3$, wherein $R^3$ has the meaning defined in claim 1 and $R^4$ is an alkyl group of one to four carbon atoms, and each reaction being followed by a conversion into another compound according to formula I, if desired.

9. A process as claimed in claim 8, wherein the diazotization reaction is carried out by reacting with an alkali metal nitrite, in the presence of an aqueous mineral acid.

10. Herbicidal composition, which comprises a compound as claimed in claim 1, together with a carrier.

11. A composition as claimed in claim 10, wherein at least two carriers are present, at least one of which is a surface-active agent.

12. Method of controlling unwanted plant growth at a locus, which comprises treating the locus with a compound as claimed in claim 1 or a composition as claimed in claim 10.

**Claims for the Contracting State: AT**

1. Herbicidal composition, which comprises a carrier, together with as active ingredient, a compound of the formula:—

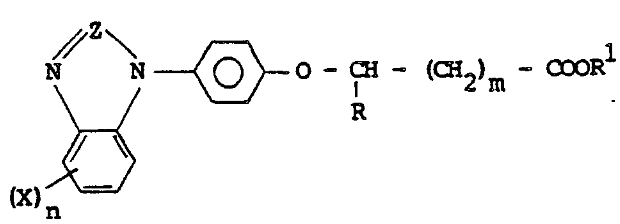

(I)

wherein Z is nitrogen or $CR^3$; X is halogen and n is 0, 1 or 2, or n is 1 and X is cyano, methyl, halomethyl, methoxy, halomethoxy, methylthio, halomethylthio, methylsulfinyl, halomethylsulfinyl, methylsulfonyl or halomethylsulfonyl; R is methyl, ethyl or methoxymethyl; m is 0, 1 or 2; $R^1$ is hydrogen, alkyl of one to four carbon atoms, an alkali metal or ammonium $(N(R^2)_4)$ ion, wherein each $R^2$ independently is hydrogen or alkyl of one to six carbon atoms; and $R^3$ is hydrogen, methyl or ethyl.

2. A composition as claimed in claim 1, wherein n = 1 and X is selected from fluoro and trifluoromethyl.

3. A composition as claimed in claim 1 or 2, wherein n = 1 or 2 and (one) X is bonded to the benzimidazole or benzotriazole moiety at the 5-position.

4. A composition as claimed in any one of claims 1 to 3, wherein R represents a methyl or ethyl group.

5. A composition as claimed in any one of claims 1 to 4, wherein $R^1$ represents hydrogen, methyl or ethyl.

6. A composition as claimed in any one of claims 1 to 5, wherein m = 0.

7. A composition as claimed in any one of the preceding claims, wherein at least two carriers are present, at least one of which is a surface-active agent.

8. Process for preparing a compound of general formula I, as defined in claim 1, characterised in that a compound of the formula:—

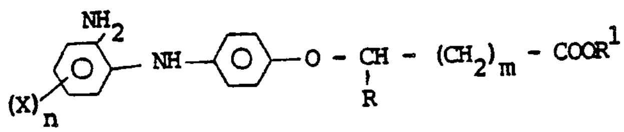
(II)

wherein n, X, R, and $R^1$ have the meanings defined in claim 1, is subjected to a cyclisation reaction.

9. A process as claimed in claim 8, wherein the compound of the formula II is converted either to a compound according to formula I wherein Z = N, by subjecting it to a diazotization reaction, or to a compound according to formula I wherein Z = $CR^3$, by subjecting it to a condensation reaction with an orthoalkanoate of formula $R^3C(OR^4)_3$, wherein $R^3$ has the meaning defined in claim 1 and $R^4$ is an alkyl group of one to four carbon atoms, and each reaction being followed by a conversion into another compound according to formula I, if desired.

10. A process as claimed in claim 9, wherein the diazotization reaction is carried out by reacting with an alkali metal nitrite, in the presence of an aqueous mineral acid.

11. Method of controlling unwanted plant growth at a locus, which comprises treating the locus with a composition as claimed in claim 1.

**Patentansprüche für die Vertragsstaaten: BE CH DE FR GB IT LI LU NL SE**

1. Eine Verbindung der Formel:

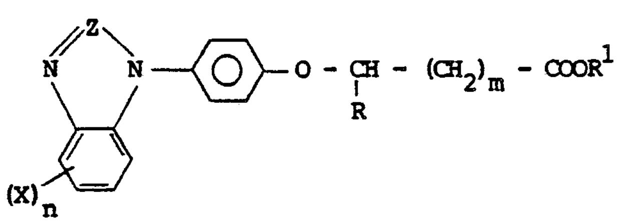
(I)

worin Z für Stickstoff oder $CR^3$ steht; X Halogen bedeutet und n den Wert 0, 1 oder 2 hat oder n den Wert 1 aufweist und X für Cyano, Methyl, Halogenmethyl, Methoxy, Halogenmethoxy, Methylthio, Halogenmethylthio, Methylsulfinyl, Halogenmethylsulfinyl, Methylsulfonyl oder Halogenmethylsulfonyl steht; R Methyl, Ethyl oder Methoxymethyl bedeutet; m den Wert 0, 1 oder 2 aufweist; $R^1$ für Wasserstoff, Alkyl mit 1 bis 4 Kohlenstoffatomen, ein Alkalimetall- oder Ammonium($N(R^2)_4$)-Ion steht, worin jeder Rest $R^2$ unabhängig voneinander Wasserstoff oder Alkyl mit 1 bis 6 Kohlenstoffatomen bedeutet; und $R^3$ Wasserstoff, Methyl oder Ethyl ist.

2. Verbindung nach Anspruch 1, worin n den Wert 1 hat und X unter Fluor und Trifluormethyl ausgewählt ist.

3. Verbindung nach Anspruch 1 oder 2, worin n den Wert 1 oder 2 hat und (ein) X in der 5-Stellung an den Benzimidazol- oder Benzotriazolrest gebunden ist.

4. Verbindung nach einem der Ansprüche 1 bis 3, worin R eine Methyl- oder Ethylgruppe bedeutet.

5. Verbindung nach einem der Ansprüche 1 bis 4, worin $R^1$ Wasserstoff, Methyl oder Ethyl darstellt.

6. Verbindung nach einem der Ansprüche 1 bis 5, worin m den Wert 0 hat.

7. Verfahren zur Herstellung einer Verbindung der allgemeinen Formel I, dadurch gekennzeichnet, daß eine Verbindung der Formel

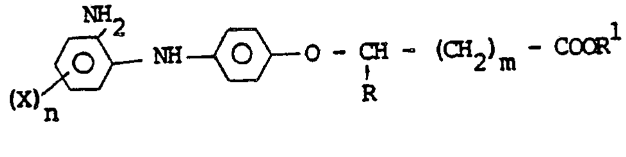
(II)

worin n, X, R und $R^1$ die in Anspruch 1 definierten Bedeutungen haben, einer Cyclisierungsreaktion unterworfen wird.

8. Verfahren nach Anspruch 7, worin die Verbindung der Formel II entweder in eine Verbindung der Formel I, worin Z für N steht, übergeführt wird, indem sie einer Diazotierungsreaktion unterworfen wird, oder in eine Verbindung der Formel I, worin Z für $CR^3$ steht, übergeführt wird, indem sie einer Kondensationsreaktion mit einem Orthoalkanoat der Formel $R^3C(OR^4)_3$, worin $R^3$ die in Anspruch 1 definierte Bedeutung hat und $R^4$ eine Alkylgruppe mit einem bis vier Kohlenstoffatomen bedeutet, unterworfen wird und auf jede Umsetzung, falls gewünscht, eine Umwandlung in eine andere Verbindung entsprechend der Formel I folgt.

9. Verfahren nach Anspruch 8, worin die Diazotierungsreaktion durch Umsetzung mit einem Alkalimetallnitrit in Gegenwart einer wässerigen Mineralsäure ausgeführt wird.

10. Herbizide Zusammensetzung, welche eine Verbindung, wie in Anspruch 1 beansprucht, zusammen mit einem Träger umfaßt.

11. Zusammensetzung nach Anspruch 10, worin wenigstens zwei Träger zugegen sind, von denen wenigstens einer ein oberflächenaktives Mittel ist.

12. Verfahren zur Bekämpfung von unerwünschtem Pflanzenwachstum an einem Ort, welches ein Behandeln des Ortes mit einer Verbindung, wie in Anspruch 1 beansprucht, oder mit einer Zusammensetzung, wie in Anspruch 10 beansprucht, umfaßt.

**Patentansprüche für den Vertragsstaat: AT**

1. Herbizide Zusammensetzung, welche einen Träger, zusammen mit einer Verbindung der Formel

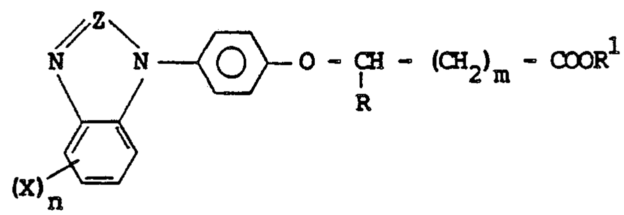

(I)

worin Z für Stickstoff oder $CR^3$ steht; X Halogen bedeutet und n den Wert 0, 1 oder 2 hat oder n den Wert 1 aufweist und X für Cyano, Methyl, Halogenmethyl, Methoxy, Halogenmethoxy, Methylthio, Halogenmethylthio, Methylsulfinyl, Halogenmethylsulfinyl, Methylsulfonyl oder Halogenmethylsulfonyl steht; R Methyl, Ethyl oder Methoxymethyl bedeutet; m den Wert 0, 1 oder 2 aufweist; $R^1$ für Wasserstoff, Alkyl mit 1 bis 4 Kohlenstoffatomen, ein Alkalimetall- oder Ammonium($N(R^2)_4$)-Ion steht, worin jeder Rest $R^2$ unabhängig voneinander Wasserstoff oder Alkyl mit 1 bis 6 Kohlenstoffatomen bedeutet; und $R^3$ Wasserstoff, Methyl oder Ethyl ist, als wirksamen Bestandteil umfaßt.

2. Zusammensetzung nach Anspruch 1, worin n den Wert 1 hat und X unter Fluor und Trifluormethyl ausgewählt ist.

3. Zusammensetzung nach Anspruch 1 oder 2, worin n den Wert 1 oder 2 hat und (ein) X in der 5-Stellung an den Benzimidazol- oder Benzotriazolrest gebunden ist.

4. Zusammensetzung nach einem der Ansprüche 1 bis 3, worin R eine Methyl- oder Ethylgruppe bedeutet.

5. Zusammensetzung nach einem der Ansprüche 1 bis 4, worin $R^1$ Wasserstoff, Methyl oder Ethyl' darstellt.

6. Zusammensetzung nach einem der Ansprüche 1 bis 5, worin m den Wert 0 hat.

7. Zusammensetzung nach einem der vorstehenden Ansprüche, worin wenigstens zwei Träger zugegen sind, von denen wenigstens einer ein oberflächenaktives Mittel ist.

8. Verfahren zur Herstellung einer Verbindung der allgemeinen Formel I, wie in Anspruch 1 definiert, dadurch gekennzeichnet, daß eine Verbindung der Formel

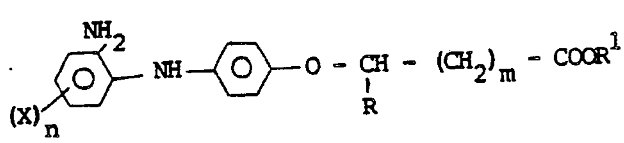

(II)

worin n, X, R und $R^1$ die in Anspruch 1 definierten Bedeutungen haben, einer Cyclisierungsreaktion unterworfen wird.

9. Verfahren nach Anspruch 8, worin die Verbindung der Formel II entweder in eine Verbindung der Formel I, worin Z für N steht, übergeführt wird, indem sie einer Diazotierungsreaktion unterworfen wird, oder in eine Verbindung der Formel I, worin Z für $CR^3$ steht, übergeführt wird, indem sie einer Kondensationsreaktion mit einem Orthoalkanoat der Formel $R^3C(OR^4)_3$, worin $R^3$ die in Anspruch 1 definierte Bedeutung hat und $R^4$ eine Alkylgruppe mit einem bis vier Kohlenstoffatomen bedeutet, unterworfen wird und auf jede Umsetzung, falls gewünscht, eine Umwandlung in eine andere Verbindung entsprechend der Formel I folgt.

10. Verfahren nach Anspruch 9, worin die Diazotierungsreaktion durch Umsetzung mit einem Alkalimetallnitrat in Gegenwart einer wässerigen Mineralsäure ausgeführt wird.

11. Verfahren zur Bekämpfung von unerwänschtem Pflanzenwachstum an einem Ort, welches ein Behandeln des Ortes mit einer Zusammensetzung, wie in Anspruch 1 beansprucht, umfaßt.

**Revendications pour les Etats contractants: BE CH DE FR GB IT LI LU NL SE**

1. Un composé de la formule:

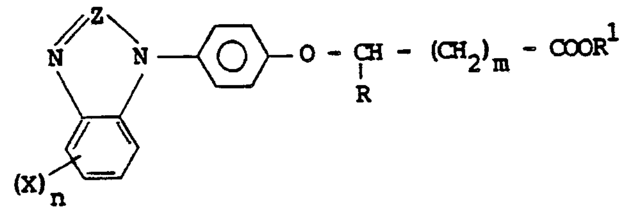

(I)

où Z est de l'azote ou $CR^3$; X est un halogène et n est 0, 1 ou 2, ou n est 1 et X est un groupe cyano, méthyle, halométhyle, méthoxy, halométhoxy, méthylthio, halométhylthio, méthylsulfinyle, halométhylsulfinyle, méthylsulfonyle ou halométhylsulfonyle; R est un groupe méthyle, éthyle ou méthoxyméthyle; m est 0, 1 ou 2; $R^1$ est de l'hydrogène, un groupe alcoyle de 1 à 4 atomes de carbone, un ion de métal alcalin ou d'ammonium $(N(R^2)_4)$, où chaque $R^2$ indépendamment est de l'hydrogène ou un groupe alcoyle de 1 à 6 atomes de carbone; et $R^3$ est de l'hydrogène ou un groupe méthyle ou éthyle.

2. Un composé selon la revendication 1, dans lequel n = 1 et X est choisi parmi le fluor et le groupe trifluorométhyle.

3. Un composé selon la revendication 1 ou 2, dans lequel n = 1 ou 2 et X (ou un X) est lié à la portion benzimidazole ou benzotriazole à la position 5.

4. Un composé selon l'une quelconque des revendications 1 à 3, dans lequel R représente un groupe méthyle ou éthyle.

5. Un composé selon l'une quelconque des revendications 1 à 4, dans lequel $R^1$ représente de l'hydrogène ou un groupe méthyle ou éthyle.

6. Un composé selon l'une quelconque des revendications 1 à 5, dans lequel m = 0.

7. Un procédé de préparation d'un composé de formule générale I, caractérisé en ce qu'un composé de la formule

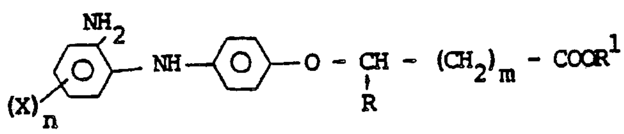

(II)

où n, X, R et $R^1$ ont les significations définies dans la revendication 1, est soumis à une réaction de cyclisation.

8. Un procédé selon la revendication 7, dans lequel le composé de formule II est transformé soit en un composé de formule I où Z = N, par exposition à une réaction de diazotation, soit en un composé de formule I où Z = $CR^3$, par exposition à une réaction de condensation avec un orthoalcanoate de formule $R^3C(OR^4)_3$, où $R^3$ a la signification définie dans la revendication 1 et $R^4$ est un groupe alcoyle de 1 à 4 atomes de carbone, chaque réaction étant suivie d'une conversion en un autre composé de formule I, si on le désire.

9. Un procédé selon la revendication 8, dans lequel on conduit la réaction de diazotation par réaction avec un nitrite de métal alcalin, en présence d'un acide minéral aqueux.

10. Une composition herbicide, qui comprend un composé selon la revendication 1, en même temps qu'un véhicule.

11. Une composition selon la revendication 10, dans laquelle au moins deux véhicules sont présents, dont l'un au moins est un agent tensio-actif.

12. Une méthode de lutte contre la pousse de plantes non désirées en un lieu, qui comprend le traitement du lieu par un composé selon la revendication 1 ou une composition selon la revendication 10.

**Revendications pour l'Etat contractant: AT**

1. Composition herbicide, qui comprend un véhicle en même temps que, comme ingrédient actif, un composé de la formule:

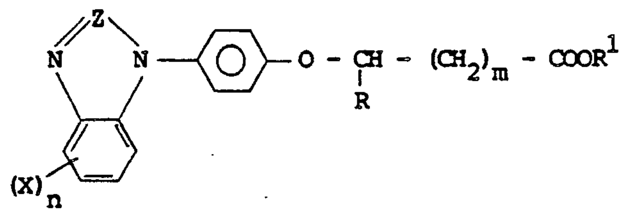

(I)

où Z est de l'azote ou $CR^3$; X est un halogène et n est 0, 1 ou 2, ou n est 1 et X est un groupe cyano, méthyle, halométhyle, méthoxy, halométhoxy, méthylthio, halométhylthio, méthylsulfinyle, halométhylsulfinyle, méthylsulfonyle ou halométhylsulfonyle; R est un groupe méthyle, éthyle ou méthoxyméthyle; m est 0, 1 ou 2; $R^1$ est de l'hydrogène, un groupe alcoyle de 1 à 4 atomes de carbone, un ion de métal alcalin ou d'ammonium ($N(R^2)_4$), où chaque $R^2$ indépendamment est de l'hydrogène ou un groupe alcoyle de 1 à 6 atomes de carbone; et $R^3$ est de l'hydrogène ou un groupe méthyle ou éthyle.

2. Une composition selon la revendication 1, dans laquelle n = 1 et X est choisi parmi le fluor et le groupe trifluorométhyle.

3. Une composition selon la revendication 1 ou 2, dans laquelle n = 1 ou 2 et X (ou un X) est lié à la portion benzimidazole ou benzotriazole à la position 5.

4. Une composition selon l'une quelconque des revendications 1 à 3, dans laquelle R représente un groupe méthyle ou éthyle.

5. Une composition selon l'une quelconque des revendications 1 à 4, dans laquelle $R^1$ représente de l'hydrogène ou un groupe méthyle ou éthyle.

6. Une composition selon l'une quelconque des revendications 1 à 5, dans laquelle m = 0.

7. Une composition selon l'une quelconque des revendications précédentes, dans laquelle au moins deux véhicules sont présents, dont l'un au moins est un agent tensio-actif.

8. Un procédé de préparation d'un composé de formule générale I, tel que défini dans la revendication 1, caractérisé en ce qu'un composé de la formule:

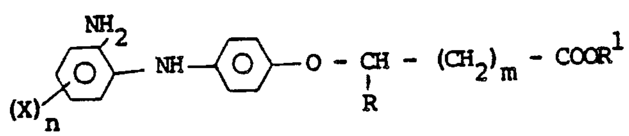

(II)

où n, X, R et $R^1$ ont les significations définies dans la revendication 1, est soumis à une réaction de cyclisation.

9. Un procédé selon la revendication 8, dans lequel le composé de formule II est transformé soit en un composé de formule I où Z = N, par exposition à une réaction de diazotation, soit en un composé de formule I où Z = $CR^3$, par exposition à une réaction de condensation avec un orthoalcanoate de formule $R^3C(OR^4)_3$, où $R^3$ a la signification définie dans la revendication 1 et $R^4$ est un groupe alcoyle de 1 à 4 atomes de carbone, chaque réaction étant suivie d'une conversion en un autre composé de formule I, si on le désire.

10. Un procédé selon la revendication 9, dans lequel on conduit la réaction de diazotation par réaction avec un nitrite de métal alcalin, en présence d'un acide minéral aqueux.

11. Une méthode de lutte contre la pousse de plantes non désirées en un lieu, qui comprend le traitement du lieu par une composition selon la revendication 1.